# EUROPEAN PATENT SPECIFICATION

(11) **EP 2 753 324 B1**
(45) Date of publication and mention of the grant of the patent: **20.12.2017**
(21) Application number: 12750390.2
(22) Date of filing: 24.08.2012
(51) Int. Cl.: A61K 31/222, C07C 233/22, A61P 9/10, A01N 1/02, C07C 233/33

(54) **LIPOPHILIC DOPAMINE DERIVATIVES AND THEIR USE**
LIPOPHILE DOPAMINDERIVATE UND IHRE VERWENDUNG
DÉRIVÉS DE LA DOPAMINE LIPOPHILE ET LEUR UTILISATION

(30) Priority: 06.09.2011 EP 11180167
(43) Date of publication of application: 16.07.2014
(73) Proprietor: Novaliq GmbH, 69120 Heidelberg (DE)
(72) Inventor: GÜNTHER, Bernhard, 69221 Dossenheim (DE); THEISINGER, Bastian, 68239 Mannheim (DE); THEISINGER, Sonja, 68239 Mannheim (DE)
(74) Representative: Cremer, Karsten
(86) International application number: PCT/EP2012/066504
(87) International publication number: WO 2013/034457

(56) References cited:
- EP-A2- 0 333 522
- WO-A1-2010/129954
- WO-A2-2009/015752
- TSAGOGIORGAS C ET AL: "N-Octanoyl-Dopamine Reduces Inflammation and Compared To Dopamine Improves Renal Function in Acute Renal Failure", TRANSPLANTATION, WILLIAMS AND WILKINS, GB, vol. 90, no. 2 supplement, 16 August 2010 (2010-08-16), page 37, XP002608348, ISSN: 0041-1337, DOI: 10.1097/00007890-201007272-00073

## Description

### BACKGROUND

The present invention relates to lipophilic derivatives of the catecholamine neurotransmitter dopamine. More specifically, it relates to the improvement in stability of these non-haemodynamic dopamine derivatives and their use in the prophylactic and/or therapeutical treatment of ischemia-related pathological conditions such as acute renal failure.

Depending on the severity of the underlying disease or injury, the mid- and long-term survival of some patients can only be ensured by organ transplantation. It is estimated that at least several ten thousand major transplantations are performed every year, with the kidneys being the most frequently transplanted organ, followed by the liver, heart, lung, and pancreas.

Transplantation involves the withdrawal of an organ or tissue from one body and its implantation into another. The success of these life-extending procedures has markedly increased over the past decades. At the same time, there are still a large number of transplantations which fail. The causes of transplant failure are often associated with (a) pre-existing diseases of the donor or pre-transplantation injury of the organ, (b) damage to the transplant after withdrawal, e.g. during transport and storage, and/or (c) transplant rejection by the immune system of the recipient.

Pre-transplantation injury of allografts such as kidneys can be induced by e.g. nephrotoxic substances which are released into the donor's body upon multiple organ failure or by the brain-death of the donor. Grafts retrieved from brain dead donors indeed show a decreased long-term survival compared to those from living donors. This kind of graft injury in brain dead donors probably occurs via several pathways, one of them relating to haemodynamic conditions, another to inflammatory responses of organs including the kidneys.

Another important cause for pre-transplantation injury of allografts is the lack of oxygen they experience upon removal from the donor's body and during storage. As soon as the cells of the allograft do not manage the shift from aerobic to anaerobic glycolysis anymore, the oxygen lack results in a critical loss of adenosine triphosphate (ATP), the primary source of energy for each cell. In addition, immune cells like macrophages or neutrophils, which are required for the prevention of inflammatory processes within the body, cannot function properly in absence of oxygen.

Hence, preservation of organs and tissues during transport and storage is absolutely vital. Typically this is achieved by keeping them in a preservation solution and cooling them to just above freezing point. A common preservation solution is based on histidine-tryptophan-ketoglutarate (HTK), aiming at the inactivation of organ function by withdrawal of extracellular sodium and calcium, together with intensive buffering of the extracellular space by means of histidine/histidine hydrochloride, so as to prolong the period during which the organs will tolerate interruption of oxygenated blood. Alternative solutions are the Euro-Collins (EC) and the University of Wisconsin (UW) solution. The latter mimics the properties of intracellular fluids, but also comprises a polymer (hydroxyethyl starch) to prevent oedema, and additives for scavenging free radicals.

It has recently been proposed, that the addition of dopamine or lipophilic dopamine derivatives to such organ perfusion and preservation mixtures can lead to the reduction of cold preservation injury (Losel RM et al., N-octanoyl dopamine, a non-haemodynamic dopamine derivative, for cell protection during hypothermic organ preservation. PLoS One. 2010 Mar 16;5(3):e9713; see also WO 2009/015752 A2).

Furthermore, it has been shown recently that a pre-treatment of donors with low-dose dopamine before kidney withdrawal has a beneficial effect on the graft function (Schnuelle P et al., Effects of donor pre-treatment with dopamine on graft function after kidney transplantation: a randomized controlled trial. JAMA. 2009 Sep 9;302(10):1067-75). Moreover, it was suggested that chemically modified dopamines, in particular N-octanoyl dopamine (NOD), may be even better suitable for donor pre-treatment than dopamine itself.

An advantage of these derivatives is that they exhibit strong reducing capacity, but at the same time they are largely devoid of haemodynamic activity and, by means of their increased lipophilicity over dopamine, have a potential for substantially increased cellular uptake.

However, it is difficult to deliver these types of lipophilic dopamine derivatives effectively. They are poorly soluble in aqueous media, and as suspensions, they cannot be injected or infused intravenously. When lipophilic dopamine derivatives are added to organ preservation solutions as such, they will partially precipitate which leads to unreliable effectiveness. The general possibility of using solubilising excipients or colloidal systems to formulate lipophilic dopamine derivatives has been proposed in WO 2009/015752, which however does not disclose any specific compositions which make use of this concept. Other documents which mention NOD are also silent about useful formulation techniques for this compound, such as Schnetze U et al., J. Cryobiol. vol. 53(3), p. 375, 2006 and Tsagogiorgas C et al., Transplantation Supplement to vol. 90 (2s), 2115, p. 37, 2010.

The need for pharmaceutical formulations that are better suitable for administration has been met recently with the development of a physiologically acceptable aqueous compositions comprising N-octanoyl dopamine (see co-pending international patent application no. PCT/EP2011/064074). While the compound is poorly compatible with a number of solubilising excipients, it has been found that certain nonionic surfactants, but also certain vesicle-forming amphiphilic lipids, may in fact be used to formulate N-octanoyl dopamine into a solubilised and stable aqueous composition. This formulation can be administered systemically or locally to a transplant donor for pre-treatment, but also to the transplant recipient for the prevention of ischaemic damage upon reperfusion. It is also used *in vitro* for the preservation of allografts in order to minimise cold preservation injury. For this purpose, it may, for example, be added to a conventional organ preservation medium.

Another potential drawback of some lipophilic dopamine derivatives is that they are chemically unstable and prone to oxidation, resulting in a higher potential risk of degradation during long-term storage as well as relatively short half-lives under physiological conditions. The latter could be partially outbalanced by a higher frequency of administration, which, however, is rather inconvenient to the patient and / or the medical staff. As an alternative, a complicated extended-release system may have to be developed for effective dosing of these derivatives, but such developments are costly as well as time consuming. Therefore, there is still a need for lipophilic dopamine derivatives that are more persistent under physiological conditions, have a longer half-life and are as more amenable to storage. This will be addressed by the present invention.

Besides its anti-oxidant properties, NOD also exerts a potent anti-inflammatory effect, which is likely attributed to the down-regulation of pro-inflammatory genes by inhibiting the nuclear factor 'kappa-light-chain-enhancer' NFκB of activated B-cells (Tsagogiorgas, C. et al., Transplantation Supplement to vol. 90 (2s), 2115, p. 37, 2010). Furthermore, NOD acts as an agonist on TRPV-1-receptors (transient receptor potential cation channel subfamily V member 1), resulting in the desensitization of this receptor type (Greffrath, W. et al., Acta Physiologica 2011; Vol. 201, Suppl.682, p. 108). This effect, in conjunction with the reported anti-inflammatory effect of NOD, is considered the reason for the improved renal function that is observed, when rats are treated with a prophylactic NOD-injection before induction of an acute renal failure (ARF).

This implies that kidney transplant donors and/or recipients who receive NOD prophylactically in order to prevent or reduce any transplantation-related graft injuries, are also to some degree protected against severe complications like acute renal failure. However, the majority of ARF events do not occur as a result of kidney transplantation. In consequence, the hospitalization and treatment of many ARF patients take place only after the onset of this pathological condition.

It is one of the objects of the present invention to overcome one or more limitations or disadvantages associated with the prior art. Still further objects will be understood in the light of the description and the patent claims.

### SUMMARY OF THE INVENTION

The present invention relates to lipophilic derivatives of the catecholamine neurotransmitter dopamine according to formula (I) and (II) for use in the treatment of a patient having developed an ischaemia-related pathological conditions.

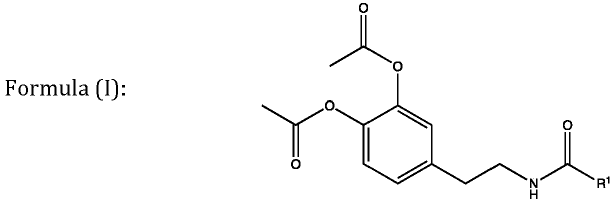

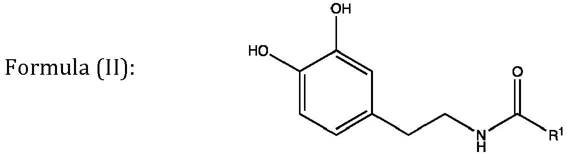

The substituent R¹ is n-heptyl. It has been found by the inventors that such compounds are, in particular, chemically and physiologically stable, thus resulting in a significantly improved half-life under physiological conditions compared to other lipophilic dopamine derivatives.

Compounds of formula (I) and (II), wherein R¹ is n-heptyl, can be applied in the treatment of patients which have already developed an ischaemia-related pathological condition. In one of the preferred embodiments, the compounds are used for the treatment of acute renal failure (ARF). In a further embodiment, they can be used in the treatment of renal, cardiac or cerebral infarctions as well as for inflammatory conditions such as myocarditis, tubulitis and/or vasculitis. For these purposes, the compounds can be administered to patients parenterally, e.g. as a continuous infusion.

In a yet further embodiment, the lipophilic dopamine derivatives of formula (I) and (II), wherein R¹ is n-heptyl for use in the treatment of a patient having developed an ischemia related pathological condition, are formulated in a pharmaceutical composition comprising an effective amount of the compound and a physiologically acceptable aqueous solvent, wherein the compound is present in a molecularly or colloidally dispersed state.

### BRIEF DESCRIPTION OF THE DRAWINGS

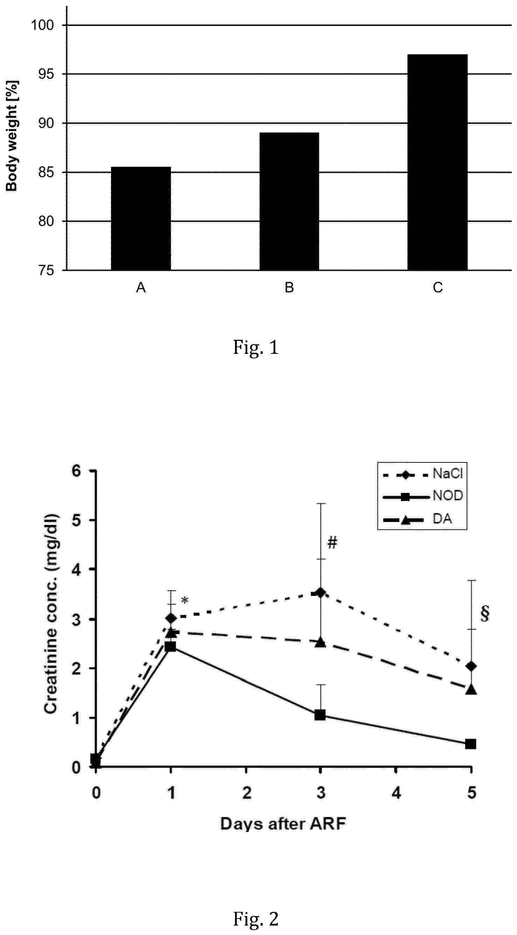

Figure 1 shows the effect of N-octanoyl dopamine (NOD) in comparison with saline and dopamine on the body weight of rats in an acute renal failure ARF model. The body weight is given in percent of the original body weight before ARF. A=saline; B=dopamine; C=NOD. The body weight of NOD-treated rats is not significantly different from 100%, in contrast to saline- or dopamine-treated rats (P<0.05).
Figure 2 shows the effect of N-octanoyl dopamine (NOD) on renal function as indicated by creatinine blood concentrations in a rat model for acute renal failure (ARF). The graphs show creatinine concentrations after treatment with saline (NaCl), dopamine (DA) and N-octanoyl dopamine (NOD) on days 1, 3 and 5 after ARF (significance levels: *NOD versus saline, P<0.05; #NOD versus both saline and dopamine, P<0.05; § NOD versus both saline and dopamine, P<0.01).

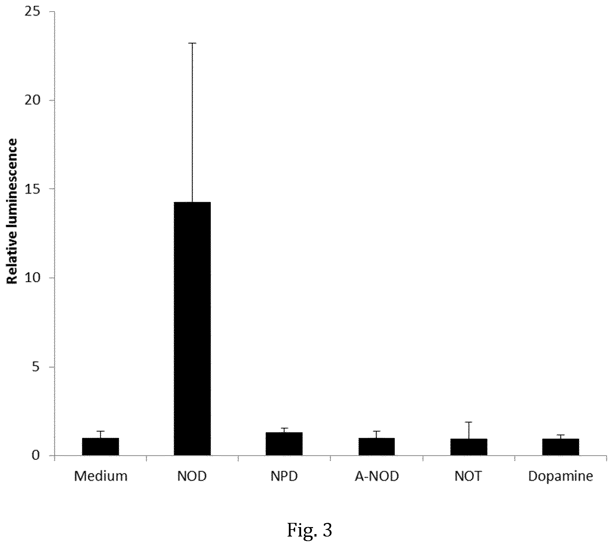

Figure 3 shows the relative effect of N-octanoyl dopamine (NOD), dopamine, N-[2-(4-hydroxyphenyl)ethyl] octanamide (referred to as NOT), N-(3,4-bisacetoxyphenylethyl)octanamide (referred to as A-NOD) and medium (control) on the level of ER stress response element (ESRE) mediated transcription of select unfolded protein response (UPR) target genes.

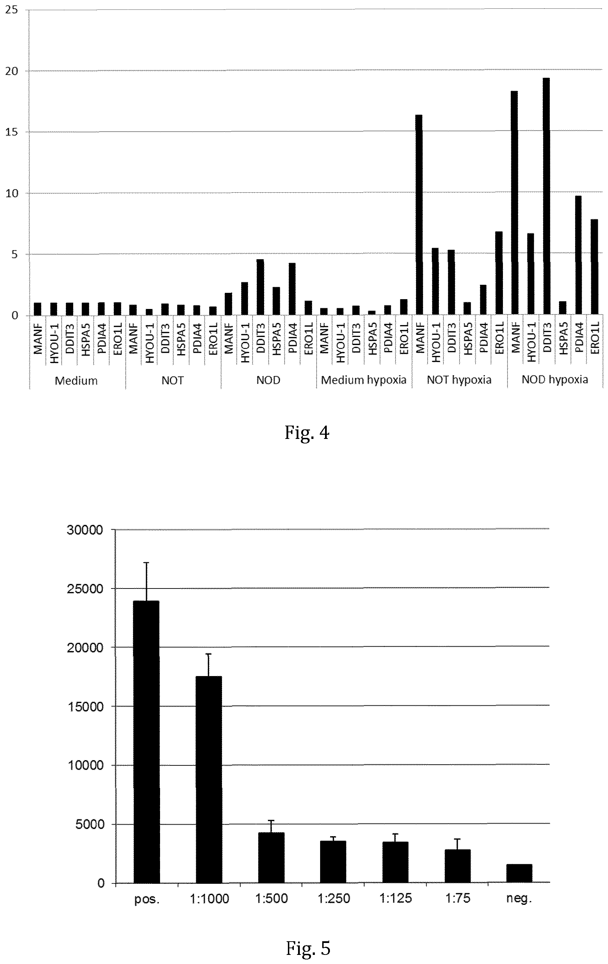

Figure 4 shows the effect of N-octanoyl dopamine (NOD) compared to N-[2-(4-hydroxyphenyl)ethyl] octanamide (NOT) and medium (control) on the level of transcription of select UPR target genes (MANF, HYOU-1, DDIT3, HSPA5, PDIA4, and ERO1L) in endothelial cells under normoxic and hypoxic conditions.
Figure 5 shows the inhibition effect of serial dilutions of N-octanoyl dopamine (NOD) on protein sulfide isomerase (PDI) in comparison to a positive (pos) and negative (neg) control.

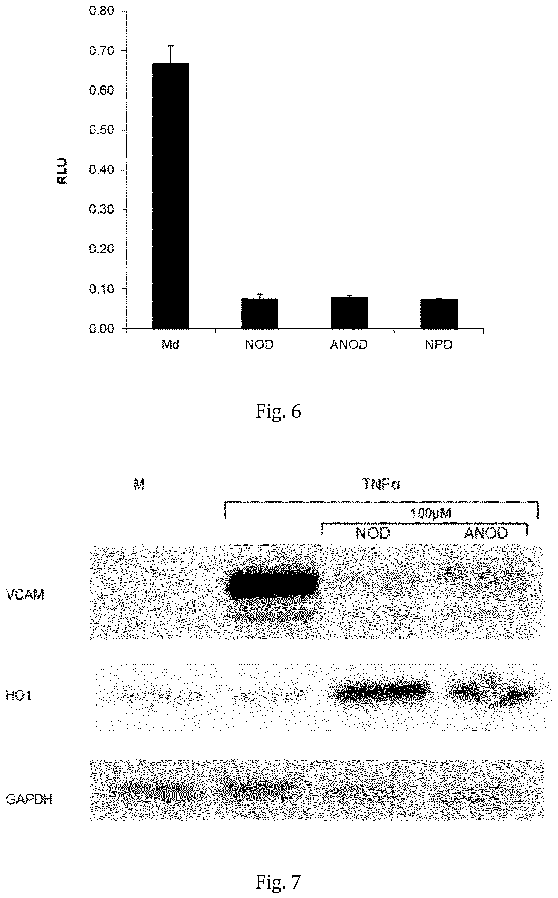

Figure 6 shows the relative hypothermic preservation properties of N-(3,4-bisacetoxyphenylethyl)octanamide (A-NOD), N-octanoyl dopamine (NOD) and N-pivaloyl dopamine (NPD) compared to a control (Md) for HUVEC (human umbilical vein endothelial cells) cells.
Figure 7 shows an immunoblot visualization depicting the binding of anti-VCAM-1 antibody and anti-HO1 antibody to lysates of cells treated with TNF-α in the presence or absence of N-(3,4-bisacetoxyphenylethyl)octanamide (A-NOD) or N-octanoyl dopamine (NOD). GADPH (Glyceraldehyde 3-phosphate dehydrogenase) refers to the loading control.

### DETAILED DESCRIPTION OF THE INVENTION

According to a first aspect of the present invention, a lipophilic derivatives of the catecholamine neurotransmitter dopamine for use in the treatment of a patient having developed an ischemia related pathological condition is provided which has the structure of formula (I), wherein R¹ is n-heptyl.

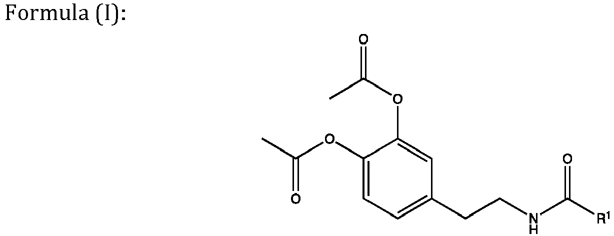

This derivative differs from dopamine (also referred to as 4-(2-aminoethyl)benzene-1,2-diol) in that the hydroxyl groups and the amino groups of dopamine have been acylated: the hydroxyl groups with acetyl residues, and the amino group with an aliphatic acyl residue having 8 carbon atoms. As used herein, the compound as defined in formula (I) include any salts, isomers, and solvates thereof.

It has been found by the inventors that this derivative is, inter alia, a useful agent for the preservation of organs and tissues. Surprisingly, it is not only superior to dopamine itself, but also has properties which are considered superior to known lipophilic dopamine derivatives such as N-octanoyl dopamine (NOD), also referred to as N-octanoyl-4-(2-aminoethyl)benzene-1,2-diol or N-octanoyl-4-2-(3,4-dihydroxyphenyl)ethylamine), in the context of particular uses.

According to the present invention R¹ is n-heptyl. This particular compound is the bisacetylated derivative of N-octanoyl dopamine (A-NOD), and may also be referred to as N-(3,4-bisacetoxyphenylethyl)octanamide or N-(3,4-bisacetatphenylethyl)octanoylamide. Its structure is depicted in formula (III). Again, any corresponding salts, isomers, and solvates are also within the scope of the invention.

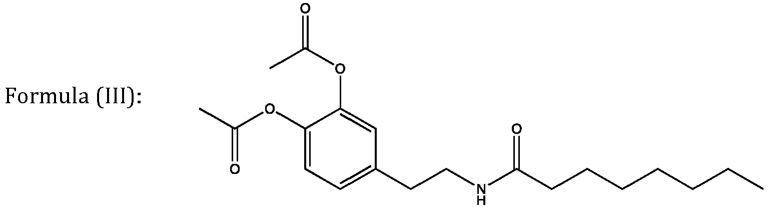

The compound may be prepared by first reacting dopamine, or a salt, isomer, or solvate thereof, with an R¹-functionalized acylating agent in order to obtain the intermediate of formula (II). As used herein, the term R¹-functionalized acylating agent includes acid halides, anhydrides, carboxylic acids and the like, which acts to deliver the R¹ n-heptyl of the compound of structure (II).

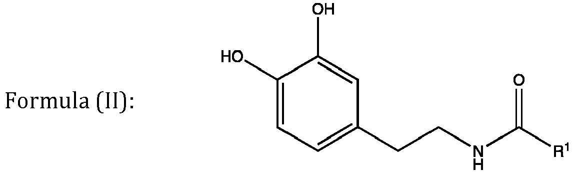

In a second step, the compounds of formula (I) may be prepared by reacting the intermediate of formula (11) with an acetylating agent. As used herein, the term acetylating agent includes anhydrides, acetyl halides, acetic acid and the like. A preferred acetylating agent is acetic anhydride.

In fact, it has been found that the compounds offer substantial improvement in their chemical stability compared to other lipophilic dopamine derivatives. The enhanced stability which also leads to an increased half-life of the compounds under physiological conditions leads to the major benefit of a prolonged physiological effect *in vivo.* This brings about two major advantages for the administration of these compounds: Firstly, the dosing frequency can be reduced, thus increasing the convenience for patients and/or medical staff. Secondly, the costly and time-consuming development of sophisticated extended release formulations which could also enhance the duration of the physiological effect can be avoided.

In one aspect, the invention provides compounds of formula (I) or formula (II) for use in the treatment of patients having developed an ischaemia-related pathological condition. The inventors have surprisingly discovered that these compounds not only prevent ischaemic damage when applied to an organ or tissue transplant, or donor or recipient thereof, but they are actually capable of reducing damage to tissues and organs after an ischaemic event has already occurred. The compounds enhance the recovery and restoration of tissues affected from ischaemic events and thus actually promote healing. This effect was entirely unexpected. No similar effect occurs with dopamine, which only acts as an agent that can preserve tissues and prevent ischaemic damage when introduced before the ischaemic event occurs.

In the context of the invention, ischaemia-related pathological conditions include any diseases, symptoms or conditions characterised by the presence of ischaemia in a tissue or organ, or by a substantially increased risk of developing an ischaemic condition.

Ischaemia, or an ischaemic condition, is a condition in which there is a restriction in blood supply to a tissue or organ. Since oxygen is mainly bound to haemoglobin in red blood cells, insufficient blood supply causes tissue to become hypoxic. Moreover, restricted blood flow leads to a shortage in oxygen and nutrients such as glucose, and to a local accumulation of metabolic wastes, all of which may contribute to tissue dysfunction, damage and necrosis. Highly aerobic organs such as the heart or the brain exhibit a pronounced sensitivity to ischaemia: in these, irreversible tissue necrosis may occur within only a few minutes after the beginning of an ischaemic event.

Ischaemia may be caused by the withdrawal of organs or tissues from the body. In one particular embodiment, this complete disruption of blood supply occurs upon removal of organs such as kidneys, heart, liver, lung, intestinal sections, pancreas as well as isolated insular cells for the purpose of transplantation.

Ischaemia may also result from a limited or interrupted blood supply because of e.g. clogged or damaged blood vessels, vasoconstriction or extensive blood loss. Irrespective of the cause, a lack of oxygen in tissues or organs leads to ischaemia-related pathological conditions.

At the cellular level, acute stress conditions causing a lack of nutrients and oxygen such as ischaemia contribute to an increase and accumulation of misfolded or unfolded proteins in the endoplasmic reticulum (ER), which is responsible for protein folding and assembly. Such defects arise due to an increase of the protein-folding load in the ER under such conditions and disruption of reactions required for correct folding. Prolonged and persistent stress can lead to eventual cell death. However, a conserved signal transduction system referred to as the unfolded protein response (UPR) has been evolved as a stress response mechanism to protect the ER and restore ER and cellular homeostasis. The triggering of UPR can lead to increase in the expression of UPR target genes of, for example, ER chaperones (e.g. the glucose-regulated proteins, or GRPS) which are responsible for assisting in the correct folding of proteins, while overall decreasing translation and reducing the production of new proteins. It has been suggested that the induction of UPR can lead to improved cell survival against ischaemia and under hypoxic conditions.

It has now been found by the inventors that N-octanoyl dopamine (NOD), in particular, can induce UPR. Genome-wide gene expression profiling of endothelial cells treated with NOD indicated an upregulation in the expression of UPR target genes compared to untreated cells. NOD significantly activates the transcription of UPR targeted genes, and is able to do so under both normoxic and hypoxic conditions. In fact, NOD induces significantly higher levels of transcription under hypoxic conditions, indicating that NOD has a positive synergistic effect on UPR transcription in combination with conditions where oxygen is low or lacking.

It is believed that NOD induces the UPR mechanism via the combination of two possible modes of action - through the inhibition of protein disulfide isomerase (PDI), and by increasing the number of reducing equivalents in the ER. Disulfide bonds are highly important to the stability of many folded protein structures. The ER lumen under normal conditions is generally oxidative and supportive towards the formation of these bonds. Molecular oxygen is used the terminal acceptor of electrons in the redox pathway through which two cysteine residues are oxidized to form a disulfide bond, with the ER-resident protein disulfide isomerase (PDI) playing a key role in the process. NOD was found to inhibit PDI in a dose-dependent manner. The disruption of the disulfide-bond forming process becomes particularly acute under hypoxic conditions and in the presence of NOD, which may account for the synergistic effect observed on UPR gene transcription under hypoxic conditions.

Hence in context of the invention, NOD can be used to help activate the cytoprotective UPR mechanism in tissues and organs. This can lead to protection against ischemic or oxidative stress damage and promote cell survival. By induction of UPR, NOD can have a useful effect in promoting restoration of cellular homeostasis before, during, as well as after ischemic events, and consequently, in a larger scale effect, prevent tissue dysfunction, damage and necrosis.

The ischaemia-related pathological conditions which may be therapeutically treated according to the invention may affect various organs or appendages including the kidneys, heart, brain, and any limbs, and the patients who receive the treatment may suffer from the respective conditions in various stages and with varying degrees of severity.

In one of the particular embodiments, the patient receiving the treatment suffers from acute kidney failure. Acute kidney failure, also known as acute kidney injury or acute renal failure, describes a condition where kidney function is rapidly lost, often in response to insufficient renal blood flow. The condition may lead to several severe complications, including metabolic acidosis, hyperkalaemia, pulmonary oedema, uraemia, substantial changes in body fluid balance, and damage to other organs.

The symptoms of acute kidney injury reflect the various aspects of loss of renal functions. The concentration of metabolites such as urea and other potentially toxic compounds in the blood rises and leads to headache, fatigue, nausea, vomiting. and loss of appetite. Cardiac function is impaired by increasing potassium concentrations which may lead to life-threatening cardiac dysrhythmia.

According to the RIFLE criteria established by the Acute Dialysis Quality Initiative (ADQI) group, patients suffering from acute kidney injury are classified into five stages: (1) "Risk": serum creatinine levels are increased at least 1.5 times or urine production is below 0.5 mL/kg body weight for 6 hours; (2) "Injury": serum creatinine levels are increased at least two-fold or urine production is below 0.5 mL/kg for 12 hours; (3) Failure: serum creatinine levels are increased at least threefold or higher than 355 µmol/L or urine production is below 0.3 mL/kg for 24 hours; (4) "Loss": persistent kidney injury or complete loss of kidney function for more than 4 weeks; (5) "End-stage renal disease": complete loss of kidney function for more than 3 months.

Preferably, the treatment according to the present invention is carried out with patients having developed acute kidney injury in stages 1 through 4. In a further embodiment, patients classified in stages 1, 2 or 3 are treated as defined herein. Preferably, treatment is commenced shortly after diagnosis.

A further medical use of the compounds of formula (I) or (11) as defined above is the treatment of patients suffering from, or having developed, an infarction, such as renal, cardiac or cerebral infarction. As used herein, an infarction describes a condition or disease which is characterised by the necrosis of a tissue or organ, usually caused by hypoxaemia. Histologically, anaemic infarctions ("white infarctions") are differentiated from haemorrhagic infarctions ("red infarctions"), depending on whether a blood flow restriction occurs upstream or downstream of the infarcted area. In anaemic infarctions (or infarcts), the restriction is typically upstream, such as a vasoconstriction of arteria or arterioles supplying the infracted organ or tissue, whereas in haemorrhagic infarctions, the venous outflow is decreased or obstructed so that substantial amounts of blood are present in the infarcted area. The treatment according to the invention may be performed with patients having either type of infarction.

Renal infarction is related to acute kidney injury as defined above, but requires that necrosis is manifest in the kidney.

Myocardial infarction is characterised by the necrosis of myocardial tissue. It results from ischaemia which affects at least a region of the heart muscle, which typically develops after an occlusion of a coronary artery. Such occlusion may occur after an atherosclerotic plaque has eroded, ruptured or detached from the arterial wall, or result from a coronary artery spasm, anaemia, arrhythmias, or severe hypotension.

Frequent symptoms of acute myocardial infarction include chest pain (angina pectoris), often described as a sensation of tightness, pressure, or squeezing, typically radiating to the left arm, but possibly also to other regions such as the neck, back, lower jaw, and the upper central region of the abdomen. Dyspnoea occurs when the damage to the heart muscle limits the output of the left ventricle, causing left ventricular failure and consequent pulmonary oedema. Other symptoms possibly resulting from the pain-induced secretion of catecholamines include sweating, nausea, vomiting, weakness, and palpitations. In severe cases, acute myocardial infarction is associated with unconsciousness resulting from inadequate cerebral blood flow, or even sudden death due to ventricular fibrillation.

Cerebral infarction is a cerebrovascular accident, or stroke, that results from cerebral ischaemia. It is typically caused by the occlusion of a blood vessel such as to substantially reduce or even interrupt the blood supply to a cerebral region, and results in the necrosis of the affected tissue. The vascular occlusion in turn may be caused by a thrombus such as formed from an arterial plaque, or a fat droplet such as may be released from the bone marrow of an injured bone, or aggregations of migrating cancer cells or bacteria.

The symptoms of cerebral infarction are largely determined by the function of the affected cerebral tissue. Contralateral hemiparesis will follow from an infarct located in the primary motor cortex. If the brainstem is affected, brainstem syndromes will be observed, such as Weber's, Benedikt, Wallenberg's, Gubler, or Millard-Gubler syndrome. Further typical effects include weakness and loss of sensation on the side of the body that is opposite to the side of the affected region of the brain, including pupil dilation, light reaction, lack of eye movement, and impairment of other reflexes. If the infarction affects the left part of the brain, speech will be slurred.

In a further particular embodiment, the treatment according to the invention is given to a patient having developed a haemorrhagic apoplectic stroke. As used herein, a haemorrhagic apoplectic stroke refers to a stroke or cerebrovascular accident caused by intracranial or intracerebral haemorrhage. Intracranial haemorrhage means that the bleeding occurs anywhere within the skull vault. Intracerebral haemorrhage, or intra-axial haemorrhage, is a subcategory thereof in that the haemorrhagic event is localised in the brain itself. Intracerebral haemorrhage in turn may be differentiated into intraparenchymal and intraventricular haemorrhage, depending on whether the bleeding occurs in the brain parenchyma or the ventricular system.

Haemorrhagic apoplectic strokes, generally speaking, may be associated with similar symptoms as other forms of stroke. In addition, they may produce typical symptoms of increased intracranial pressure, such as headache, vomiting without nausea, back pain, ocular palsies, papilledema, and decreased level of consciousness.

In a further embodiment, the patient receiving the therapy according to the invention is affected by an inflammatory condition which may result in ischaemia, such as myocarditis, tubulitis, or vasculitis.

As used herein, myocarditis refers to any inflammation of heart muscle except in response to the occlusion of a coronary artery. It may be due to an infection caused by a virus, bacterium, fungus, protozoon, or parasite; autoimmune reactions, or as a hypersensitivity reaction to therapeutic or other drugs. Tubulitis is an inflammatory condition affecting the renal tubules of the nephron. Vasculitis refers to any inflammation of blood vessels, whether venous (phlebitis) or arterial (arteritis).

For application to an organ or tissue, compounds according to formula (I) with R¹ being defined as above are typically formulated as liquid compositions using a suitable liquid carrier and any further components as may be required.

For the administration to humans such as organ donors or patients, compounds according to formula (I) or formula (11) with R¹ being defined as above may be administered by any route which will lead to the biological availability of the compound at the target site. In particular, the compounds or the composition thereof may be administered orally or parenterally.

For oral administration, the compounds may, for example, be formulated as tablets, hard capsules, or softgels, using common pharmaceutical excipients as known to the person skilled in the art.

Parenteral administration, as used herein, refers to any invasive type of administration by injection or infusion, including intravenous, intraarterial, subcutaneous, intramuscular, locoregional, intraluminal, and intradermal administration. In a preferred embodiment, the route is selected from intravenous, intraarterial, subcutaneous, and intramuscular administration. Presently most preferred is intravenous administration, in particular in the form of a continuous infusion.

In a particular embodiment, the use of the compounds as described herein involves the administration of a pharmaceutical composition comprising an effective amount of the compound and a physiologically acceptable aqueous solvent. In the composition, the compound is present in a molecularly or colloidally dispersed state.

An effective amount means an amount appropriate for achieving the effect within the context of the intended use. The effective amounts of a particular compound may, for example, differ between organ preservation solutions, donor pre-treatment injections, and therapeutic infusions.

A physiologically acceptable aqueous solvent is water or an aqueous solution of compounds, in particular pharmaceutical excipients, which are considered safe with respect to the incorporated amount and the intended use. For example, the aqueous solvent may be sterile isotonic sodium chloride solution, or a sterile buffer solution.

The composition may further comprise a physiologically acceptable amphiphilic excipient, in particular a surfactant, which may be used as a pharmaceutical excipient in that it is safe and well-tolerated at least at the incorporated level and in view of the intended use, taking into consideration the route and frequency of administration.

The molecularly dispersed state refers to a true molecular solution. In a liquid solution, the molecules of the solute(s) are individually solvated and surrounded by solvent molecules. In contrast, the colloidally dispersed state means that a material, in this a compound as defined by formula (I) or (II), is present in structures having a colloidal size, i.e. they are substantially larger than the respective molecules but too small to be visible to the unaided eye. Colloids typically have a diameter of between approximately 1 and 500 nanometres. (H. Stricker, Physikalische Pharmazie, 3rd Edition, page 440). Therefore, colloidal structures are practically not visible with a light microscope and do not result in market turbidity of the solution, but rather in opalescence.

Colloidal structures of various types are known to exist in different types of colloidal liquids. In isotropic colloidal solutions, the properties of the solution are the same regardless of the direction of measurement. In other words, in the isotropic state, all directions are indistinguishable from each other. For example, a micellar solution may be isotropic. In anisotropic colloidal solutions, there is orientation and/or alignment of molecules which causes the physical properties of the solution to vary for different directions of measurement. Such anisotropic solutions are referred to as liquid crystals, or liquid-crystalline phases, or mesophases.

In one of the preferred embodiments, the composition comprises the compound, i.e. active ingredient, in this colloidally dispersed state. The colloidal particles that are dispersed in the aqueous solvent will typically contain both the amphiphilic excipient and the active ingredient. As used herein, colloidal dispersion may also be referred to as a colloidal solution.

According to the further preferred embodiment, the composition comprises an amphiphilic excipient selected from the group of nonionic surfactants. Pharmaceutically acceptable nonionic surfactants include, for example, tyloxapol, poloxamers such as poloxamer 188, poloxamer 407, Pluronic F68LF or Lutrol F68, Pluronic F127, Pluronic L-G2LF and Pluronic L62D, polysorbates such as polysorbate 20, polysorbate 60, and polysorbate 80, polyoxyethylene castor oil derivatives, sorbitan esters, polyoxyl stearates, and mixtures of two or more thereof. In a specific embodiment, the nonionic surfactant is a polysorbate. In a further specific embodiment, the nonionic surfactant is polysorbate 80.

Optionally, the composition comprises two or more amphiphilic excipients or surfactants. In one of the preferred embodiments, two or more nonionic surfactants are used in combination. For example, a polysorbate, such as polysorbate 20 or 80, may be combined with Cremophor EL or Cremophor RH. In a further embodiment, the composition comprises a nonionic surfactant in combination with an ionic surfactant such as a phospholipid.

The amphiphilic excipient or surfactant, or combination of surfactants, may be incorporated in such an amount that it forms micelles in which the active compound is solubilised. Generally speaking, micelles are colloidal aggregates of amphiphilic molecules in a solvent. They may be spherical, but can also have very different shapes. In an aqueous system, a typical spherical micelle comprises surfactant molecules whose hydrophilic moieties are in contact with the surrounding solvent, sequestering the hydrophobic molecular regions in the micelle centre. Poorly water-soluble lipophilic compounds may be dissolved in the core of such micelles.

In order for micelle formation to occur, the concentration of the surfactant (or surfactants) must be above the critical micelle concentration (CMC). Therefore, the amount of surfactant in the composition should be selected above this concentration if a micellar solution is aimed at. Moreover, the amount of surfactant should be selected sufficiently high as to solubilise the incorporated amount of active compound. At the same time, the amount of surfactant must be low enough to avoid undesirable effects in the donor of a transplant, the transplant itself, or the recipient of the transplant.

In one particular embodiment, the amount of amphiphilic excipient is at least about 0.05 wt.-%. According to a further embodiment, the amount is from about 0.1 wt.-% to about 30 wt.-%, or from about 0.5 wt.-% to about 20 wt.-%, respectively. However, if the composition is to be used as a concentrate to be added to a commercial solution for tissue and organ preservation, the concentrate itself may also comprise the amphiphilic excipient at a relatively high concentration, taking into account the dilution factor.

In a further preferred embodiment, the composition is free of organic solvents or co-solvents such as ethanol, glycerol, propylene glycol, or polyethylene glycol. According to another preferred embodiment, the composition may contain small amounts of such solvents or co-solvents, such as up to about 2 wt.-%.

The composition may comprise further inactive pharmaceutical ingredients as required or appropriate. For example, it may comprise one or more excipients for adjusting the tonicity of the formulation. It is preferred that the composition is adapted to exhibit an osmotic pressure of roughly 310 mOsmol/kg, such as in the range from about 200 to about 450 mOsmol/kg, or in the range from about 250 to about 400 mOsmol/kg, or in the range from about 280 to about 350 mOsmol/kg, respectively. If the intended use is the preservation of allografts during storage and transport, it should be ensured that, optionally after dilution with a conventional organ preservation solution, the composition has a physiological osmolality of about 300 to 330 mOsmol/kg. Suitable excipients for adjusting the osmotic pressure include, for example, salts, sugars, sugar alcohols, and amino acids. Among the salts, buffer salt or sodium chloride are particularly suitable. Useful sugars and sugar alcohols include, for example, glucose, raffinose, trehalose, sorbitol, and mannitol, to mention only a few.

Moreover, the composition may comprise one or more excipients for adjusting the pH value, which is preferably selected in the range from about pH 3 to about pH 8. More preferably, the pH is not higher than about 7, such as from about pH 4 to about pH 7.0, or from about pH 4.5 to about pH 6.5. Suitable excipients for adjusting the pH include physiologically acceptable organic or inorganic acids, bases, and buffer salts. The latter salts may at the same time function as physiological electrolytes, such as salts of sodium, potassium, magnesium, and calcium.

The composition may further comprise one or more stabilisers, such as complexing or chelating agents like EDTA, and/or antioxidants such as vitamin E or vitamin E derivatives, ascorbic acid, sulphites, hydrogen sulphites, gallic acid esters, butyl hydroxyanisole, butyl hydroxytoluene or acetylcysteine; viscosity-increasing agents such as water-soluble polymers; preservatives (in case the composition is to be packaged in multiple-dose containers and used for parenteral administration); lactobionic acid, allopurinol, glutathione, adenosine; amino acids such as histidine, tryptophan, glutamic acid, aminoglutamic acid, or ketoglutarate.

Optionally, the invention may be carried out by formulating a powder or liquid concentrate from which a composition as described herein can be reconstituted. For example, for achieving an extended shelf life it may be useful to formulate the solid components of the composition as a sterile lyophilised powder which may, prior to its use, be dissolved or dispersed in an appropriate aqueous carrier or diluent. Alternatively, a liquid concentrate may be formulated which, upon dilution with an aqueous medium, yields the final composition to be used for transplant donor pre-treatment, allograft preservation, or treatment of transplant recipients. Such liquid concentrate not only has the advantage of having a low weight and volume which makes it easier to manufacture, transport, store, and handle it, but also provides an opportunity to depart from physiological parameters such as pH or osmolality during storage, e.g. with an eye on an extended shelf life. The physiological properties required for its use are then achieved by appropriately diluting the concentrate.

In another embodiment, the composition is in the form of a microemulsion. As used herein, a microemulsion is a clear, thermodynamically stable, optically isotropic mixture of a lipophilic component, a hydrophilic component, and an amphiphilic component. Typically, a microemulsion forms spontaneously when the components are combined and mixed with each other, without requiring high energy input as is normally required for the formation of an "ordinary" emulsion. Microemulsions may have a colloidal lipophilic phase dispersed in a hydrophilic phase, or a hydrophilic phase colloidally dispersed in a lipophilic phase. The size of the dispersed phases is usually in the range from about 5 nm to about 400 nm, and most often below about 200 nm. In one of the preferred embodiments of the invention, the particle size is from about 5 nm to about 100 nm. In terms of its rheological properties, the microemulsion may be in the form of a liquid or a gel, i.e. in liquid or semisolid form. In a preferred embodiment, the microemulsion is in liquid form. If a microemulsion is used, the lipophilic component is preferably selected from excipients which are per se suitable for parenteral use. For example, a highly purified triglyceride oil or semi-synthetic medium-chain triglycerides may be used.

In a further embodiment, the amphiphilic excipient is a vesicle-forming phospholipid. In this case, the composition is designed as a colloidal dispersion of liposomes, wherein the liposomes incorporate the active compound. As used herein, a liposome is a vesicle formed from at least one bilayer, wherein the bilayer is composed of aggregated (or assembled) amphiphilic lipids. The bilayer exhibit some similarity with biological membranes in that it is hydrophilic towards the inside and outside of the vesicle, whereas the lipophilic region is sandwiched in between these hydrophilic regions. Larger liposomes often have two or more concentric bilayers. Small liposomes tend to be rather spherical, but larger vesicles may exist in various shapes.

Depending on the selected preparation method and manufacturing conditions, the resulting liposomes may be described as multilamellar vesicles (MLV), small unilamellar vesicles (SUV), or large unilamellar vesicles (LUV). MLVs differ from SUVs and LUVs in that MLVs have two or more lipid bilayers. Hence, MLVs appear useful in particular for being loaded with lipophilic drug substances which dissolve in, or associate with, the lipophilic regions of the vesicle membranes. In contrast, SUVs and LUVs are especially useful for the encapsulation of hydrophilic compounds within the aqueous compartment of the liposomes. Typically, MLVs have a diameter from about 200 nm up to several microns. SUVs typically range from about 80 nm to about 200-300 nm, whereas LUVs are normally understood to be larger than about 200-300 nm in average. Within the context of the invention, the diameters are understood as z-averages as measured with laser diffraction or photon correlation spectroscopy. In the context of the present invention, colloidal liposomes should be used, and very large MLVs may not fall into this category.

The amphiphilic lipids from which the liposomes are composed typically include at least one phospholipid. Phospholipids are amphiphilic lipids comprising a phosphate group, which is negatively charged and thus substantially hydrophilic. Phospholipids may be classified as glycerophospholipids (or phosphoglycerides, characterised by the presence of a glyceryl moiety) or phosphosphingolipids (or ceramides, such as sphingomyelin). Liposomes may contain native, semisynthetic and/or synthetic phospholipids.

Typically, liposomes comprise at least one glycerophospholipid (or phosphoglyceride). Such glycerophospholipids are in fact the most commonly used vesicle-forming lipids in liposomes. Commonly used glycerophospholipids include those which are derived from native lecithins, such as soy or egg lecithin, or from the (partial) hydration products thereof. Lecithins contain high amounts of phosphatidylcholines, but may also comprise smaller amounts of phosphoric acid, choline, fatty acids, glycerol, glycolipids, triglycerides, phosphatidylethanolamines, and phosphatidylinositol. Phosphatidylcholines are glycerophospholipid that comprise choline as a head group, in contrast to phosphatidylethanolamines and phosphatidylglycerols.

In phosphatidylcholines, two hydroxyl group of the glyceryl residue are linked via ester bonds to acyl groups, which are typically derived from medium to long chain fatty acids. Common acyl groups in phosphatidylcholines (but also in phosphatidylethanolamines and phosphatidylglycerols) used as constituents of liposomes include myristoyl, palmitoyl, stearoyl, and oleoyl groups.

Due to the negative charge of the phosphate group and the positive charge of the choline, phosphatidylcholines are always zwitterionic (sometimes also referred to as neutral). Phosphatidylethanolamines are also zwitterionic over large pH-ranges, but can exist as anions in basic environments. Phosphatidylglycerols are anionic.

Besides one or more glycerophospholipids, liposomes may comprise one or more lipids which are themselves not capable of forming bilayers, but which modify or stabilise such bilayers. An example of such membrane-modifying lipid is cholesterol.

Methods for the preparations and characterization of liposomes and liposome preparations are known as such to the skilled person. Often, multilamellar vesicles will form spontaneously when amphiphilic lipids are hydrated, whereas the formation of small unilamellar vesicles usually requires a process involving substantial energy input, such as ultrasonication or high pressure homogenization. Further methods for preparing and characterizing liposomes have been, for example, described by S. Vemuri et al. [Preparation and characterization of liposomes as therapeutic delivery systems: a review. Pharm Acta Helv. 1995, 70(2):95-111].

Of the known liposomes, those which may be used according to the invention have a predominantly colloidal size, i.e., their average particle size lies below about 500 nm. Also preferred is a diameter of up to about 300 nm, or not higher than 200 nm, respectively. Such average particle size will usually allow sterile filtration through a filter with a pore size of 0.22 µm, which is a significant advantage in case the composition is not stable enough to withstand heat sterilization.

As mentioned, the composition of the invention may be used in the treatment of a patient having developed an ischemia-related pathological condition.

Further embodiments will become obvious from the following examples which illustrate the invention in some of its major aspects.

### EXAMPLES

### Example 1: Preparation of N-octanoyl dopamine (NOD)

Step 1: Ethyl chloroformate (69.1 mmol) was added carefully over a period of 10 minutes under vigorous stirring to a solution of octanoic acid (69.3 mmol), i-Pr₂NEt (69.6 mmol) in 90 mL of anhydrous THF. The resulting mixture was stirred at room temperature (RT) for approximately 3 h and then 50 mL ethyl acetate and 100 mL water was added. The organic phase was separated, dried over anhydrous MgSO₄ and concentrated. The resulting crude mixed anhydride product was used in the next step without further purification.

Step 2: Dopamine hydrochloride (66 mmol) was dissolved in 50 mL of DMF. A solution of the mixed anhydride solution in 50 mL of ethyl acetate was added dropwise over a period of 20 minutes. An additional equivalent of *i*-Pr₂NEt (66 mMol) was then added to the resulting cloudy reaction mixture, which was stirred over night at room temperature and under darkness. To the reaction mixture was then added 200 mL of aqueous NaHCO₃ (5 w/w%) and aqueous sodium sulphite (1 w/w%). The organic phase was separated and the aqueous phase was extracted with two portions of ethyl acetate. The combined organic phases were washed sequentially with brine, 0.5 M aqueous H₂SO₄, then brine, dried over MgSO₄, filtered and concentrated. Recrystallisation twice from dichloromethane afforded N-octanoyl dopamine (NOD) as a white powder in 58 % yield; m.p. 67 - 67.9 °C (heating rate: 1 °C/min).

### Example 2: Preparation of N-(3,4-bisacetoxyphenethyl)octanamide

N-octanoyl dopamine (25 mMol) and sodium acetate (30.5 mMol) were added to 30 mL of acetic anhydride. The reaction mixture was stirred at 80 °C for 3 h, then poured onto 100 mL of ice-cold water and stirred for 10 minutes. The mixture was filtered and the precipitate was washed with three portions of ice-cold water. Recrystallisation twice from Et₂O afforded *N*-(3,4-bisacetoxyphenylethyl)octanamide in 65 % yield; m.p. 76 °C (heating rate: 1 °C/min).

### Example 3: Effect of N-octanoyl dopamine on body weight in ARF rat model

Kidneys of Lewis rats (n=8) were clamped for 45 min to induce acute renal failure (ARF). Afterwards the rats were treated with continuous infusions (10 µL/h over 5 days) of either saline, dopamine or N-octanoyl dopamine (NOD), using an osmotic minipump. The concentration of dopamine and NOD was 40 µmol/mL. The rats' body weight was monitored for 5 days. While the saline- and dopamine-treated rats lost ∼14 % and ∼11 % of their body weight due to the ARF, there was no significant change in the weights before and after the ARF for the NOD-treated rats, as shown in figure 1.

### Example 4: Effect of N-octanoyl dopamine on renal function in ARF rat model

Kidneys of Lewis rats (n=8) were clamped for 45 min to induce acute renal failure (ARF). Afterwards the rats were treated with continuous infusions (10 µL/h over 5 days) of either saline, dopamine or N-octanoyl dopamine (NOD), using an osmotic minipump and the same concentrations as in Example 3. The rats' renal function, as measured by the serum creatinine concentration, was monitored for 5 days. In result, NOD-treated rats showed a significantly improved renal function from day 1 and 3 compared to saline-treated and dopamine-treated rats, respectively (P<0.05), as shown in figure 2.

### Example 5: Effect of N-octanoyl dopamine on renal histology

The kidneys of the Lewis rats of examples 3 and 4 were harvested. The renal tissue was examined with regard to the infiltration by inflammatory ED1-cells and signs of necrotic tubulus cells. In result, less necrotic tissue and a significantly reduced number of ED1-cells were observed for NOD-treated rats in comparison with saline- and dopamine-treated rats (P<0.01). Moreover, it was found that the TNF-α activity in the renal tissue as measured by PCR was significantly reduced in the case of NOD-treated rats (P<0.01).

### Example 6: Effect of NOD on ESRE-mediated UPR gene transcription

The induction of UPR is mediated by the binding of transcription factors to an ER stress response element (ESRE) featured in the UPR (unfolded protein response) target genes. Endothelial cells were transfected by means of lentiviral transfection using a reporter luciferase construct under the control of ESRE responsive elements. After two days, the transfected cells were stimulated for 24 hours with NOD or dopamine or NPD (N-pivaloyl dopamine, also referred to as N-[2-(3,4-dihydroxyphenyl)ethyl] *tert*-butylamide), or N-[2-(4-hydroxyphenyl)ethyl] octanamide (referred to as NOT), or medium (control). Chemiluminescence activity of the luciferase was then measured. It was found that NOD was able to strongly induce ESRE-mediated transcription (as shown in Fig. 3) in endothelial cells compared to dopamine and NOT. As expected, its diacetylated derivative A-NOD did not significantly induce transcription, which is believed to be due to experimental conditions such that hydrolysis of the acetyl groups to free the reactive o-hydroxy functionality, unlike *in vivo*, did not effectively occur.

### Example 7: Quantification of UPR gene transcription of NOD, NOT, and benzoic acid derivative treated endothelial cells.

**Table 1 Relative fold-increase of transcription compared to untreated endothelial cells**

| 2ΔΔ Ct | NOD | NOT | BB | BBNB | BBNO |
|---|---|---|---|---|---|
| MANF | 4.53 | 1.08 | 1.03 | 0.99 | 0.96 |
| DDIT3 | 37.80 | 1.12 | 1.01 | 0.90 | 3.74 |
| HYOU1 | 5.17 | 1.26 | 0.90 | 0.84 | 4.72 |
| HSPA5 | 11.40 | 0.94 | 0.60 | 0.65 | 1.08 |
| 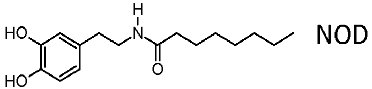 | | | 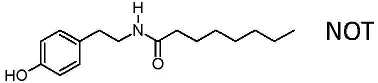 | | |
| 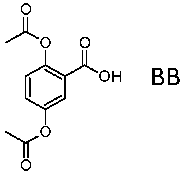 | 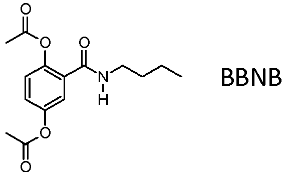 | | 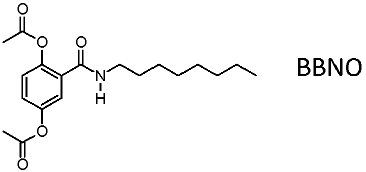 | | |

Endothelial cells were stimulated for 24 h with 100 µM of NOD, NOT, BB, BBNB or BBNO (see Table 1 for compound structures). RNA was isolated from each sample and transcribed into cDNA. Expression of selected UPR target genes was assessed by qPCR. Quantification of the transcription of these genes revealed that NOD has a greater upregulatory influence on the selected UPR genes compared to NOT and benzoic acid derivatives BB, BBNB and BBNO.

### Example 8: UPR gene transcription of endothelial cells under normoxic and hypoxic conditions in the presence of NOD or NOT

Endothelial cells were cultured for 24 h under normoxic conditions in the presence or absence of 100 µM of NOD or NOT. Cells that were cultured in the absence of either compounds (medium) served as a control. In addition, the cells were cultured under hypoxic conditions for 24 h in the presence or absence of 100 µM NOD or NOT. RNA was isolated and transcribed into cDNA. The expression of a selected set of UPR target genes was assessed by qPCR. As depicted in Fig. 4, only NOD induced UPR gene transcription under normoxic conditions. Interestingly, hypoxic conditions alone (medium, hypoxia) did not induce UPR gene transcription, but provided a synergistic effect on UPR gene.

### Example 9: Protein Disulfide Isomerase (PDI) Inhibition by NOD

Modulation of PDI activity by NOD was examined by using a fluorescence-based commercially available ProteoStat™ PDI assay kit and according to the kit protocol. The protocol was applied to serial dilutions of NOD, which were measured for inhibition of PDI activity using a fluorescent microplate reader at an excitation setting of about 500 nm and an emission filter of about 603 nm. As depicted in Fig. 5, NOD inhibits PDI in a dose-dependent manner.

### Example 10: Hypothermic Preservation Properties of A-NOD, NOD and NPD.

The hypothermic preservation properties of NOD, A-NOD and N-pivaloyl dopamine (also referred to as NPD, or N-[2-(3,4-dihydroxyphenyl)ethyl] *tert-*butylamide) were examined. Human umbilical vein endothelial cells (HUVEC) were seeded in 24 well plates and grown until confluence. Hereafter, HUVEC were stimulated for 1 h with 100 µM of NOD, A-NOD or NPD. Cells that were not treated (Md) served as control. The medium was aspirated and the cells were stored for 24 hrs on ice in University of Wisconsin (UW) solution. Cell damage was assessed by measuring lactate dehydrogenase (LDH) release, using a commercially available LDH release assay (Roche). To this end, 100 µl of supernatant was added to 100 µl of assay reaction mixture. The plates were incubated for 30 minutes and the absorption (OD₄₉₀) was assessed. All experimental conditions were performed in triplicate.

An increase in LDH activity correlates with the amount of dead or damaged cells. As shown in Fig. 6, LDH activity measured from the A-NOD, NOD and NPD treated cells were significantly lower compared to the untreated cell control (Md).

### Example 11: Effect of A-NOD and NOD on the inhibition of TNF-α mediated VCAM-1 expression

The anti-inflammatory effects of A-NOD and NOD were examined, specifically as to their impact on TNF-α (tumour necrosis factor alpha) mediated VCAM-1 (vascular cell adhesion molecule 1) expression. Human umbilical vein endothelial cells (HUVEC) were cultured until confluence. Hereafter the cells were stimulated for 24 h with TNF-α in the presence or absence of 100 µM NOD or A-NOD. Cells that were not stimulated (M) served as control. Cells were harvested and lysed in Laemli buffer. Hereafter, 20 µL of the lysates were boiled and subjected to SDS-PAGE. The resulting gel was blotted on PVF membranes and the membrane was subsequently incubated overnight with TBS/Tween/5% dry milk powder to block unspecific binding. The upper part of the membrane was probed with an anti-VCAM-1 antibody and the lower part with an anti-HO1 antibody (heme oxygenase-1). After thoroughly washing the membrane was incubated with appropriate HRP conjugates, bands were visualized using chemoluminescence (shown in Fig. 7). The membrane was stripped and subsequently probed with and GAPDH monoclonal to demonstrate equal loading of the gel.

Cells treated with TNF-α, but in the absence of A-NOD or NOD showed strong anti-VCAM-1 antibody binding in comparison to the cells co-treated with A-NOD or NOD. Cells treated with A-NOD and NOD showed anti-HO1 antibody binding. HO1, or heme oxygenate-1 enzyme, has anti-inflammatory functions.

## Claims

1. A compound of formula (I) or formula (11) wherein R¹ is n-heptyl, 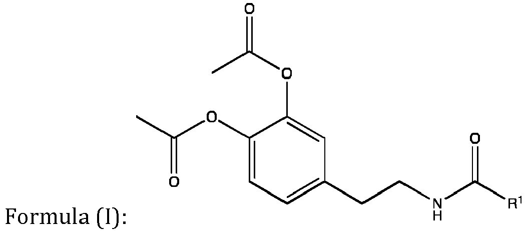 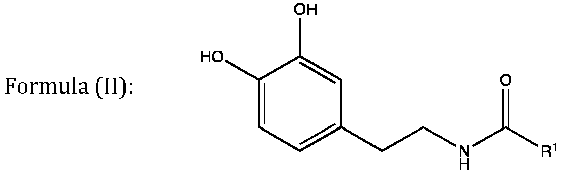 for use in the treatment of a patient having developed an ischemia-related pathological condition.

2. The compound for use according to claim 1, wherein the ischemia-related pathological condition is acute renal failure.

3. The compound for use according to claim 1, wherein the ischemia-related pathological condition is selected from renal, cardiac and cerebral infarction.

4. The compound for use according to claim 1, wherein the ischemia-related pathological condition is a haemorrhagic apoplectic stroke.

5. The compound for use according to claim 1, wherein the ischemia-related pathological condition is selected from myocarditis, tubulitis, and/or vasculitis.

6. The compound for use according to claims 1 to 5, wherein the compound is administered parenterally.

7. The compound for use according to claim 6, wherein the compound is administered by continuous infusion.

8. The compound for use according to claims 1 to 7, comprising the administration of a pharmaceutical composition comprising:
(a) an effective amount of the compound, and
(b) a physiologically acceptable aqueous solvent;
wherein the compound is present in a molecularly or colloidally dispersed state.

## Patentansprüche

1. Eine Verbindung der Formel (I) oder Formel (II), wobei R¹ n-Heptyl ist, 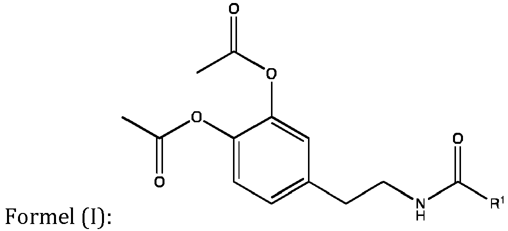 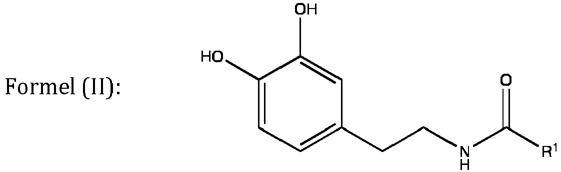 zur Verwendung bei der Behandlung eines Patienten, der einen ischämiebedingten pathologischen Zustand entwickelt hat.

2. Die Verbindung zur Verwendung gemäß Anspruch 1, wobei der ischämiebedingte pathologische Zustand akutes Nierenversagen ist.

3. Die Verbindung zur Verwendung gemäß Anspruch 1, wobei der ischämiebedingte pathologische Zustand ausgewählt ist aus Niereninfarkt, Herzinfarkt und Hirninfarkt.

4. Die Verbindung zur Verwendung gemäß Anspruch 1, wobei der ischämiebedingte pathologische Zustand ein hämorrhagischer Hirnschlag ist.

5. Die Verbindung zur Verwendung gemäß Anspruch 1, wobei der Ischämiebedingte pathologische Zustand ausgewählt ist aus Myokarditis, Tubulitis, und/oder Vasculitis.

6. Die Verbindung zur Verwendung gemäß den Ansprüchen 1 bis 5, wobei die Verbindung parenteral verabreicht wird.

7. Die Verbindung zur Verwendung gemäß Anspruch 6, wobei die Verbindung durch kontinuierliche Infusion verabreicht wird.

8. Die Verbindung zur Verwendung gemäß den Ansprüchen 1 bis 7, umfassend die Verabreichung einer pharmazeutischen Verbindung umfassend:
(a) eine wirksame Menge der Verbindung, und
(b) eine physiologisch akzeptable wässrige Lösung;
wobei die Verbindung in einem molekular oder kolloidal dispergierten Zustand vorliegt.

## Revendications

1. Composé de la formule (I) ou de la formule (11) dans laquelle R' est un n-heptyle, 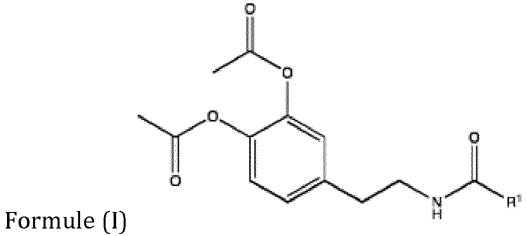 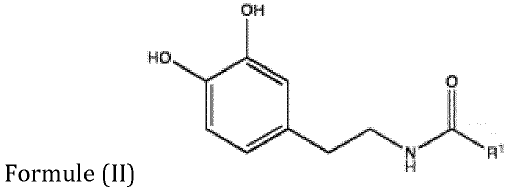 ledit composé étant utilisé dans le traitement d'un patient ayant développé un état pathologique lié à l'ischémie.

2. Composé destiné à être utilisé selon la revendication 1, l'état pathologique lié à l'ischémie étant une insuffisance rénale aiguë.

3. Composé destiné à être utilisé selon la revendication 1, l'état pathologique lié à l'ischémie étant choisi parmi l'infarctus rénal, cardiaque et cérébral.

4. Composé destiné à être utilisé selon la revendication 1, l'état pathologique lié à l'ischémie étant un accident vasculaire cérébral hémorragique.

5. Composé destiné à être utilisé selon la revendication 1, l'état pathologique lié à l'ischémie étant choisi parmi la myocardite, la tubulite et/ou la vascularite.

6. Composé destiné à être utilisé selon les revendications 1 à 5, le composé étant administré par voie parentérale.

7. Composé destiné à être utilisé selon la revendication 6, le composé étant administré par perfusion continue.

8. Composé destiné à être utilisé selon les revendications 1 à 7, comprenant l'administration d'une composition pharmaceutique comprenant :
(a) une quantité efficace du composé, et
(b) un solvant aqueux physiologiquement acceptable ;
le composé étant présent dans un état de dispersion moléculaire ou colloïdale.
